# EUROPÄISCHE PATENTANMELDUNG

(11) **EP 0 645 356 A1**
(43) Veröffentlichungstag der Anmeldung: **29.03.1995**
(21) Anmeldenummer: 94114253.1
(22) Anmeldetag: 10.09.1994
(51) Int. Cl.: C07C 13/20, C07C 5/11

(54) **Verfahren zur Herstellung von Cyclohexen durch partielle Hydrierung von Benzol**

(30) Priorität: 20.09.1993 DE 4331839
(71) Anmelder: BASF Aktiengesellschaft, D-67063 Ludwigshafen (DE)
(72) Erfinder: Wulff-Doering, Joachim, Dr., D-67227 Frankenthal (DE); Polanek, Peter, Dr., D-69469 Weinheim (DE)

(57) **Zusammenfassung**

Verfahren zur Herstellung von Cyclohexen durch partielle Hydrierung von Benzol mit Wasserstoff in Gegenwart von Wasser und Rutheniumkatalysatoren bei erhöhter Temperatur in flüssiger Phase, indem man als Katalysatorträger ZnO einsetzt und man der wäßrigen Phase eine Base zusetzt.

## Beschreibung

Die vorliegende Erfindung betrifft Verfahren zur Herstellung von Cyclohexen durch partielle Hydrierung von Benzol mit Wasserstoff an Rutheniumkatalysatoren bei erhöhter Temperatur in wäßriger, basischer Phase, wobei der Katalysatorträger ZnO ist.

Aus der US-A-4 197 415 ist ein Verfahren zur Herstellung von cyclischen Olefinen durch partielle Hydrierung von aromatischen Kohlenwasserstoffen in Gegenwart von Rutheniumkatalysatoren bekannt. Als Katalysatoren werden Tränkkatalysatoren auf Trägern wie Mordenit, die als Promotoren Phosphate von Metallen der 2. bis 8. Nebengruppe des Periodensystems der Elemente enthalten, eingesetzt. Mit einem Ruthenium/Zinkkatalysator auf einem zeolithischen Träger werden hierbei Cyclohexenausbeuten von 1 mol-% mit einer Selektivität von 7 % erhalten. Diese Werte sind verbesserungsbedürftig.

Aus der EP-A 55 495 ist bekannt, daß man cyclische Alkene, z.B. Cyclohexen durch partielle Gasphasenhydrierung von aromatischen Kohlenwasserstoffen wie Benzol in Gegenwart eines Rutheniumkatalysators unter Mitverwendung von Wasserdampf erhalten kann. Neben Ruthenium kann der Katalysator ein oder mehrere Metallverbindungen aus der Reihe Eisen, Chrom, Germanium, Blei, Zink, Nickel und vorzugsweise Natrium enthalten. Die Hydrierung in der Gasphase hat den Nachteil, daß große Reaktionsräume erforderlich sind und somit die Raumzeitausbeute sinkt.

Aus der US-A-3 912 782 ist ein Verfahren zur Herstellung von Cyclohexen durch Hydrierung von Benzol im saurem bzw. neutralem pH-Bereich in Gegenwart von Rutheniumkatalysatoren, die Mangan, Kobalt oder Nickel als Promotoren enthalten, bekannt. Hierbei werden Cyclohexenausbeuten bis zu 20 % bei einer Selektivität von 34 % erzielt.

Aus JP 60 184 031 ist die partielle Hydrierung von aromatischen Kohlenwasserstoffen mit Ruthenium/Zink-Mischoxiden zu den entsprechenden Cycloolefinen bekannt. In Gegenwart von alkalifreiem Wasser werden Cyclohexenausbeuten von bis zu 20 % bei Selektivitäten von 34 % bei einer Reaktionsdauer von 3 h erreicht. Sowohl die erreichten Cyclohexenausbeuten als auch die lange Reaktionszeit sind verbesserungswürdig.

Der vorliegenden Erfindung lag daher die Aufgabe zugrunde, den zuvor genannten Nachteilen abzuhelfen.

Demgemäß wurde ein neues und verbessertes Verfahren zur Herstellung von Cyclohexen durch partielle Hydrierung von Benzol mit Wasserstoff in Gegenwart von Wasser und Rutheniumkatalysatoren bei erhöhter Temperatur in flüssiger Phase, welches dadurch gekennzeichnet ist, daß man als Katalysatorträger ZnO einsetzt und man der wäßrigen Phase eine Base zusetzt.

Das erfindungsgemäße Verfahren läßt sich wie folgt durchführen:
Man kann den Ruthenium-Katalysator, Wasser, Benzol und die Base in einer Druckapparatur vorlegen und Temperaturen von 20 bis 300°C, bevorzugt 70 bis 250°C, besonders bevorzugt 100 bis 200°C unter Rühren einstellen. Nach Erreichen der jeweiligen Reaktionstemperatur kann Wasserstoff mit 1 bis 350 bar aufgepreßt werden. Man hält während der Reaktion in der Regel einen Wasserstoffpartialdruck von 1 bis 200 bar, bevorzugt 5 bis 150 bar, besonders bevorzugt 10 bis 100 bar ein, wobei die Hydrierung in der Regel bevorzugt in flüssiger Phase erfolgt, d.h. die Temperatur- und Druckbedingungen sollten so aufeinander abgestimmt sein, daß die Einhaltung einer flüssigen Phase gewährleistet wird. Ferner ist bevorzugt, daß die Wassermenge bezogen auf die Gesamtflüssigkeitsmenge 5 bis 90 Gew.-%, bevorzugt 10 bis 85 Gew.-%, besonders bevorzugt 25 bis 75 Gew.-% beträgt und daß die wäßrige Phase alkalisch ist. Die Hydrierung kann sowohl diskontinuierlich als auch kontinuierlich, z.B. in Suspension oder mit einem fest angeordnetem Katalysator durchgeführt werden.

Als Rutheniumkatalysatoren eignen sich solche, die Ruthenium auf einem ZnO-Träger enthalten, wobei der ZnO-Träger in der Regel 0,01 bis 50 Gew.-%, bevorzugt O,02 bis 25 Gew.-%, besonders bevorzugt 0,5 bis 10 Gew.-%, insbesondere 0,1 bis 10 Gew.-% Ruthenium enthält. Ganz besonders bevorzugt sind solche Rutheniumkatalysatoren, die aus einem ZnO-Träger und in der Regel 0,01 bis 50 Gew.-%, bevorzugt O,02 bis 25 Gew.-%, besonders bevorzugt 0,5 bis 10 Gew.-%, insbesondere 0,1 bis 10 Gew.-% Ruthenium bestehen.

Der Katalysator kann durch Adsorptionstränkung, Tränkung oder auch durch Fällung hergestellt werden. Als besonders geeignet hat sich dabei die Diffusionstränkung erwiesen. Geeignete Rutheniumverbindungen sind beispielsweise Rutheniumhalogenide, Rutheniumnitrate, Rutheniumhydroxide und Rutheniumoxide, besonders bevorzugt Rutheniumhalogenide, besonders bevorzugt Ruthenium-(III)-chlorid. Diese Rutheniumverbindungen können z.B. in Lösung mit dem ZnO-Träger unter Rühren 1 bis 10 Stunden bei einer Temperatur von 0 bis 100°C, bevorzugt 10 bis 70°C, besonders bevorzugt 20 bis 40°C, insbesondere bei Raumtemperatur, gemischt werden. Hierbei erhält man den gewünschtem Katalysator, wobei man den Abschluß der Diffusionstränkung an der Entfärbung der Lösung erkennen kann. Der erhaltene Katalysator kann von der Lösung abdekantiert oder abfiltriert, mit Wasser gewaschen und anschließend bei 50 bis 150°C, vorzugsweise bei 70 bis 95°C, getrocknet werden.

Die Reduktion des Katalysators kann unter einem Wasserstoffpartialdruck von 1 bis 100 bar, insbesondere 1 bis 20 bar und einer Temperatur von 50 bis 400°C, insbesondere einer Temperatur von 120 bis 350°C, für einen Zeitraum von 4 bis 10 Stunden, vorzugsweise 4 bis 6 Stunden erfolgen. Der so erhaltene Katalysator kann ohne weitere Behandlung für die partielle Benzolhydrierung genutzt werden.

Die erfindungsgemäße Umsetzung wird in der Regel in Gegenwart von Wasser durchgeführt, wobei die Wassermenge, bezogen auf die Gesamtflüssigkeitsmenge, in der Regel vorzugsweise 5 bis 90 Vol.-%, bevorzugt 10 bis 80 Vol.-% beträgt.

Als Basen eignen sich Alkalihydroxide, Erdalkalihydroxide und Amine, bevorzugt Alkalihydroxide und Erdalkalihydroxide, besonders bevorzugt Alkalihydroxide wie Lithiumhydroxid, Natriumhydroxid und Kaliumhydroxid, bevorzugt Natriumhydroxid und Kaliumhydroxid.

Das erfindungsgemäße Verfahren hat den Vorteil, daß es mit verbesserten Raumzeitausbeuten verläuft und höhere Umsätze und Selektivitäten erzielt werden. Ein weiterer Vorteil besteht in den kürzeren Verweilzeiten.

Das nach dem Verfahren der Erfindung hergestellte Cyclohexen eignet sich zur Herstellung von Cyclohexanol, einem wichtigen Ausgangsstoff für die Herstellung von Faservorprodukten.

### Beispiele

### Beispiel 1

0,85 g RuCl₃ * 3 H₂O wurden in 3 l Wasser gelöst. Die erhaltene wäßrige Lösung hatte einen Rutheniumgehalt von 0,3 Gew.-%. In dieser wäßrigen Lösung wurden 30 g Zinkoxidpulver suspendiert, welches als Träger diente. Die erhaltene Mischung wurde 3 h gerührt. Danach konnte das Pulver sich absetzen. Die Abscheidung des Rutheniums auf dem Träger wurde durch die Entfärbung der ursprünglich tief braunen Farbe des gelösten Rutheniums bestätigt. Die Suspension wurde abfiltriert und mit Wasser gewaschen. Der erhaltene Rückstand wurde über Nacht (ca. 16 h) bei 80°C getrocknet. Anschließend wurde das Pulver bei 350°C innerhalb von 6 h in einem Wasserstoffstrom reduziert. Der erhaltene Katalysator hat einen Gehalt an metallischen Ruthenium von 1,0 Gew.-% bezogen auf den Träger.

1,2 g dieses Katalysators wurden mit 93 ml Wasser, 47 ml Benzol und 2,3 g NaOH in einen 0,3 l-Autoklaven gefüllt und unter Rühren auf 150°C aufgeheizt. Nach Erreichen der Temperatur wurde Wasserstoff aufgepreßt, so daß der Reaktionsdruck von 50 bar über die Reaktionsdauer von 30 min aufrecht gehalten wurde.

Hiernach wurde der Autoklav entspannt und der Inhalt zügig abgekühlt. Die organische Phase wurde abgetrennt und gaschromatographisch analysiert. Als Ergebnis der Hydrierung wurde ein Umsatz an Benzol von 10,5 % bei einer Cyclohexenselektivität von 85,7 erhalten (Cyclohexenausbeute 9,0 %). Neben Cyclohexen und Benzol wurde nur Cyclohexan als Nebenprodukt gefunden.

### Beispiel 2 bis 4

Die Hydrierung wurde mit dem gleichen Katalysator und unter den gleichen Reaktionsbedingungen wie in Beispiel 1 durchgeführt, außer daß die Reaktionszeiten, wie in Tabelle 1 angegeben, geändert wurden.

**Tabelle 1**

| Hydrierergebnisse mit unterschiedlicher Reaktionsdauer | | | |
|---|---|---|---|
| Reaktionszeit | Umsatz | Cyclohexenselektivität | Cyclohexenausbeute |
| [min] | [%] | [%] | [%] |
| 60 | 25,2 | 77,0 | 19,4 |
| 90 | 34,7 | 70,9 | 24,6 |
| 120 | 47,5 | 64,8 | 30,8 |

Die Ergebnisse zeigen, daß mit längeren Reaktionszeiten hohe Cyclohexenausbeuten bei guter Cyclohexenselektivität erhalten werden können.

### Vergleichsbeispiel 1

Die Hydrierung wurde mit dem gleichen Katalysator und unter den gleichen Reaktionsbedingungen wie in Beispiel 1 durchgeführt, außer daß auf die Zugabe von NaOH verzichtet wurde.

Das Ergebnis war ein Benzolumsatz von 74,3 % und eine Cyclohexenausbeute von 11,6 % (Selektivität 15,6 %).

Der Versuch zeigt, daß die Zugabe von NaOH notwendig ist, um sowohl hohe Cyclohexenausbeuten als auch hohe Cyclohexenselektivitäten zu erreichen.

### Vergleichsbeispiel 2

Die Hydrierung wurde mit dem gleichen Katalysator und unter den gleichen Reaktionsbedingungen wie in Beispiel 1 durchgeführt, außer daß statt NaOH eine äquimolare Menge an NaCl dem Reaktionsgemisch zugesetzt wurde.

Die Hydrierung lieferte bei einem Benzolumsatz von 45,3 % eine Cyclohexenausbeute von 12,6 % (Cyclohexenselektivität 27,8 %).

Dieses Ergebnis zeigt, daß die Alkalinität des Reaktionsgemisches notwendig ist, um Cyclohexen in hohen Ausbeuten und Selektivitäten zu erhalten.

### Vergleichsbeispiel 3

Die Hydrierung wurde mit dem gleichen Katalysator und unter den gleichen Reaktionsbedingungen wie in Beispiel 1 durchgeführt, außer daß dem Reaktionsgemisch zusätzlich 23 mg Zinkoxid zugesetzt wurde.

Die Hydrierung lieferte mit 10,1 % Benzolumsatz und 9,0 % Cyclohexen (Cyclohexenselektivität 87,1 %) die gleichen Ergebnisse wie ohne Zugabe von Zinkoxid. Auf die Zugabe von ZnO zum Reaktionsgemisch kann also verzichtet werden.

### Beispiel 4 und 5

Die Hydrierung wurde mit dem gleichen Katalysator und unter den gleichen Reaktionsbedingungen wie in Beispiel 1 durchgeführt, außer daß NaOH durch äquimolare von LiOH bzw. KOH ersetzt wurden. Die Ergebnisse sind in Tabelle 2 dargestellt.

**Tabelle 2**

| Einfluß der Art des Alkalihydroxids | | | |
|---|---|---|---|
| Alkalihydroxid | Umsatz | Cyclohexenselektivität | Cyclohexenausbeute |
| | [%] | [%] | [%] |
| KOH | 19,7 | 76,1 | 15,0 |
| LiOH | 5,8 | 87,1 | 5,1 |

Die Ergebnisse zeigen, daß auch mit anderen Alkalihydroxiden als NaOH Cyclohexen in hohen Selektivitäten gebildet wird.

### Beispiele 6 und 7

Die Hydrierung wurde mit dem gleichen Katalysator und unter den gleichen Reaktionsbedingungen wie in Beispiel 1 durchgeführt, außer daß die NaOH-Menge wie in Tabelle 3 angegeben verändert wurde.

**Tabelle 3**

| Einfluß der Menge an Natriumhydroxid | | | |
|---|---|---|---|
| NaOH | Umsatz | Cyclohexenselektivität | Cyclohexenausbeute |
| [g] | [%] | [%] | [%] |
| 1,15 | 37,9 | 61,7 | 23,4 |
| 4,6 | 2,1 | 90,5 | 1,9 |

Die Ergebnisse zeigen den Einfluß der NaOH-Menge. Durch zu hohe NaOH-Mengen geht der Umsatz stark zurück, ohne daß sich die Cyclohexenselektivität wesentlich erhöht. Mit geringeren NaOH-Mengen kann der Umsatz gesteigert werden, wobei allerdings die Cyclohexenselektivität abnimmt.

### Beispiel 8 und 9

Die Hydrierung wurde mit dem gleichen Katalysator und unter den gleichen Reaktionsbedingungen wie in Beispiel 1 durchgeführt, außer daß die Katalysatormengen entsprechend den Angaben in Tabelle 4 geändert wurden.

**Tabelle 4**

| Einfluß der Katalysatormenge | | | |
|---|---|---|---|
| Kat.-Menge | Umsatz | Cyclohexenselektivität | Cyclohexenausbeute |
| [g] | [%] | [%] | [%] |
| 2,4 | 29,9 | 72,2 | 21,6 |
| 0,6 | 9,0 | 85,6 | 7,7 |

Die Ergebnisse zeigen, daß eine Erhöhung der Katalysatormenge zu deutlich höheren Cyclohexenausbeuten bei sinkenden Selektivitäten führt. Die Verringerung der Katalysatormenge hat nur geringe Umsatzverluste bei gleicher Selektivität zur Folge.

### Beispiele 1 und 11

Es wurden mehrere Katalysatoren wie in Beispiel 1 beschrieben hergestellt, nur daß durch Konzentrationserhöhung der Ru*C13-Lösung der Rutheniumgehalt der Katalysatoren erhöht wurde. Die Hydrierung wurde unter den gleichen Reaktionsbedingungen wie in Beispiel 1 durchgeführt. Die Ergebnisse sind in Tabelle 5 dargestellt.

**Tabelle 5**

| Einfluß der Ru-Konzentration des Katalysators | | | |
|---|---|---|---|
| Ru-Menge | Umsatz | Cyclohexenselektivität | Cyclohexenausbeute |
| [%] | [%] | [%] | [%] |
| 2,5 | 17,4 | 76,4 | 13,3 |
| 5,0 | 22,4 | 75,4 | 16,9 |

Die erhaltenen Ergebnisse zeigen, daß sich durch Erhöhung der Ru-Konzentration auf dem Katalysator die Cyclohexenausbeute steigern läßt.

## Patentansprüche

1. Verfahren zur Herstellung von Cyclohexen durch partielle Hydrierung von Benzol mit Wasserstoff in Gegenwart von Wasser und Rutheniumkatalysatoren bei erhöhter Temperatur in flüssiger Phase, dadurch gekennzeichnet, daß man als Katalysatorträger ZnO einsetzt und man der wäßrigen Phase eine Base zusetzt.

2. Verfahren zur Herstellung von Cyclohexen durch partielle Hydrierung von Benzol nach Anspruch 1, dadurch gekennzeichnet, daß man die Umsetzung bei Temperaturen von 20 bis 300°C durchführt.

3. Verfahren zur Herstellung von Cyclohexen durch partielle Hydrierung von Benzol nach Anspruch 1, dadurch gekennzeichnet, daß man die Umsetzung mit einem Wasserstoffpartialdruck von 10 bis 100 bar durchführt.

4. Verfahren zur Herstellung von Cyclohexen durch partielle Hydrierung von Benzol nach Anspruch 1, dadurch gekennzeichnet, daß der Ruthenium-Katalysator 0,1 bis 10 Gew.-% Ruthenium auf einem ZnO-Träger enthält.

5. Verfahren zur Herstellung von Cyclohexen durch partielle Hydrierung von Benzol nach Anspruch 1, dadurch gekennzeichnet, daß der Ruthenium-Katalysator aus einem ZnO-Träger mit einem Rutheniumgehalt von 0,1 bis 10 Gew.-% besteht.

6. Verfahren zur Herstellung von Cyclohexen durch partielle Hydrierung von Benzol nach Anspruch 1, dadurch gekennzeichnet, daß die Wassermenge, bezogen auf die Gesamtflüssigkeitsmenge 5 bis 90 Gew.-% beträgt.

7. Verfahren zur Herstellung von Cyclohexen durch partielle Hydrierung von Benzol nach Anspruch 1, dadurch gekennzeichnet, daß die wäßrige Phase alkalisch ist.

8. Verfahren zur Herstellung von Cyclohexen durch partielle Hydrierung von Benzol nach Anspruch 1, dadurch gekennzeichnet, daß man 0,1 bis 10 Gew.-% einer Base, bezogen auf die Wassermenge einsetzt.

9. Verfahren zur Herstellung von Cyclohexen durch partielle Hydrierung von Benzol nach Anspruch 1, dadurch gekennzeichnet, daß man als Base Alkalihydroxide einsetzt.
